# EUROPEAN PATENT APPLICATION

(11) **EP 2 918 579 A1**
(43) Date of publication of application: **16.09.2015**
(21) Application number: 14159828.4
(22) Date of filing: 14.03.2014
(51) Int. Cl.: C07D 333/12, C07D 333/54, C07D 409/10

(54) **Synthesis of 2-arylmethyl-5-aryl-thiophene**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention provides a new synthetic process for the production of 2-arylmethyl-5-aryl-thiophene, an intermediate in the synthesis of C-aryl glycosides.

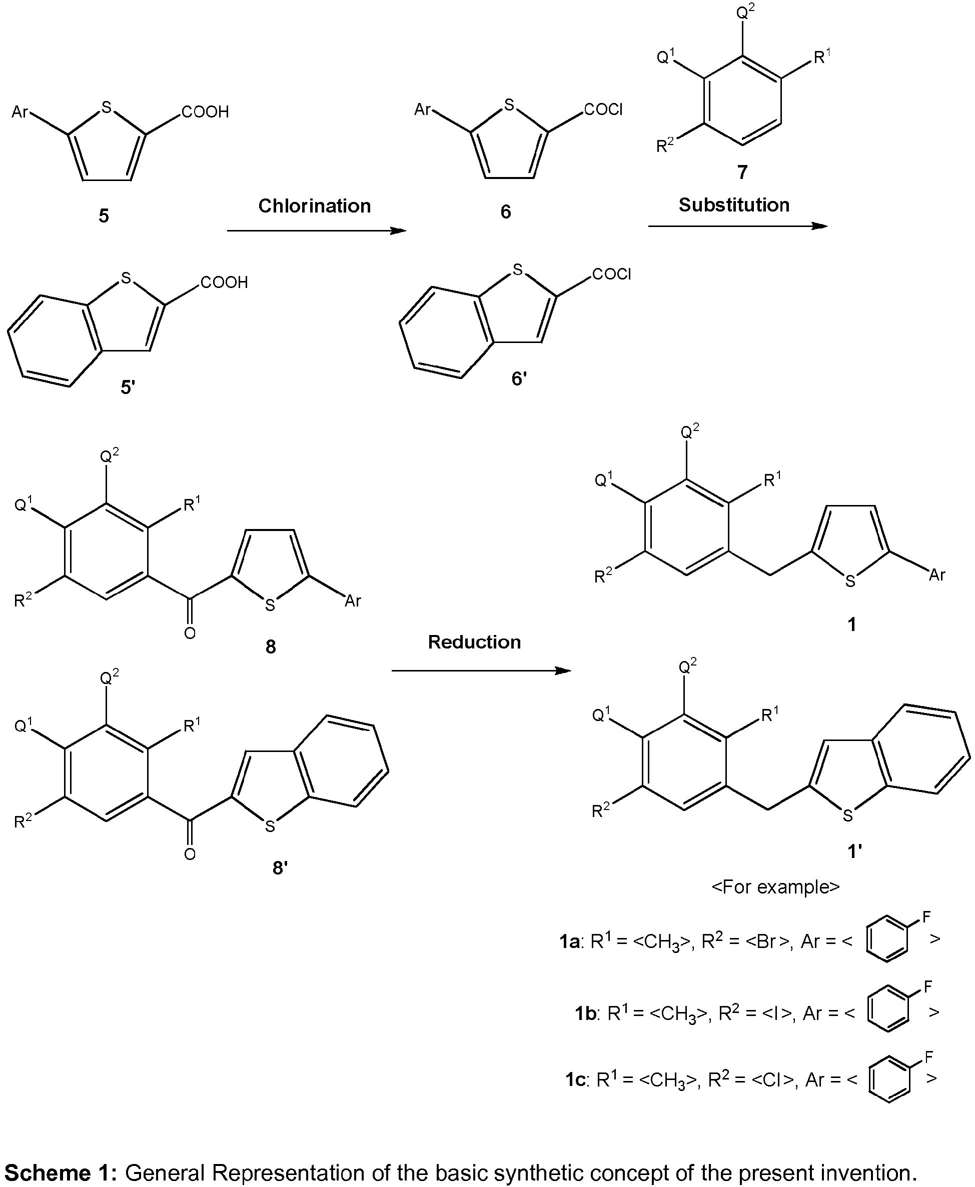

## Description

### Field of the Invention

This invention relates to a new and advantageous process for the synthesis of 2-arylmethyl-5-aryl-thiophene, which can be used as an intermediate in the synthesis of C-aryl glycosides.

### Background of the Invention

C-Aryl glycosides comprise a glucose ring moiety that is arylated at C1, as shown below:

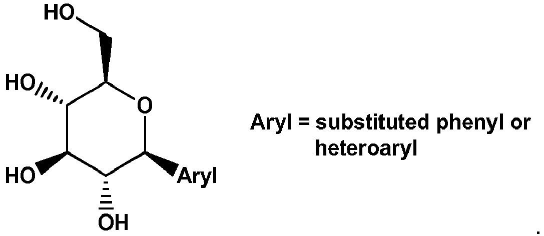

Some C-aryl glycosides have known to be an inhibitor of subtype 2 sodium-glucose transport protein (SGLT2) used for the normalization of plasma glucose levels. Exemplary C-aryl glycosides SGLT2 inhibitor may be canagliflozin ((2*S*,3*R*,4*R*,5*S*,6*R*)-2-{3-[5-[4-fluoro-phenyl)-thiophen-2-ylmethyl]-4-methyl-phenyl}-6-hydroxymethyl-tetrahydro-pyran-3,4,5-triol) having a following formula I

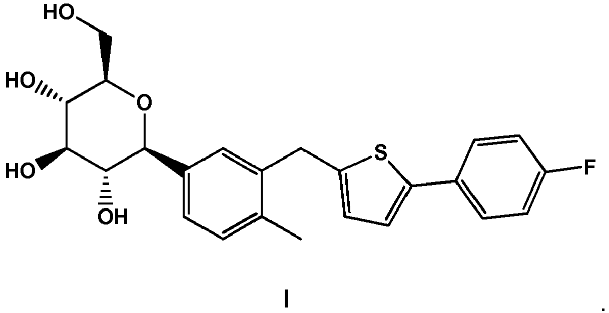

Canagliflozin and its preparation processes are disclosed in WO 2005/012326 A1, according to which 2-(5-bromo-2-methyl-benzyl)-5-(4-fluoro-phenyl)-thiophene is obtained from starting materials of 2-(4-fluoro-phenyl)-thiophene and 5-bromo-2-methyl-benzoic acid via 2-(5-bromo-2-methyl-benzoyl)-5-(4-fluoro-phenyl)-thiophene intermediate.

Similarly, WO 2012/160218 A1 describes processes for the preparation of 2-(5-bromo-2-methyl-benzyl)-5-(4-fluoro-phenyl)-thiophene from 5-bromo-2-methyl-benzoyl chloride and 2-(4-fluorophenyl)-thiophene.

WO 2010/043682 A1 describes that 2-(4-fluoro-phenyl)-thiophene is prepared by coupling 2-bromo-thiophene with 4-fluoro-phenylmagnesium bromide in the presence of nickel or palladium catalyst. 2-(4-Fluoro-phenyl)-thiophene is then reacted with 5-iodo-2-methyl-benzoic acid derivate to give 2-(5-iodo-2-methyl-benzyl)-5-(4-fluoro-phenyl)-thiophene.

The preparation of 2-(5-bromo-2-methyl-benzyl)-5-(4-fluoro-phenyl)-thiophene is also described in WO 2011/079772 A1, according to which 2-(5-bromo-2-methyl-benzoyl)-5-(4-fluoro-phenyl)-thiophene is prepared by coupling 3-chloro-3-(4-fluoro-phenyl)-propenal with 2-chloro-1-(2-methyl-5-bromo-phenyl)-ethanone in the presence of sodium sulphide, followed by reducing the resulting 2-(5-bromo-2-methyl-benzoyl)-5-(4-fluoro-phenyl)-thiophene with triethylsilane to give 2-(5-bromo-2-methyl-benzyl)-5-(4-fluoro-phenyl)-thiophene.

The above processes have disadvantages in terms of employing expensive materials and/or long processes.

In the synthesis of C-aryl glycosides such as canagliflozin of formula **I**, aryl moiety such as 2-arylmethyl-5-aryl-thiophene may represent major portion of the drug molecule and thus carry most of the cost burden. Therefore, there remains a need for shorter and more efficient process for the preparation of aforementioned intermediates in order to bring the overall synthesis of C-aryl glycosides to the most affordable level.

### Summary of the Invention

Various aspects, advantageous features and preferred embodiments of the present invention as summarized in the following items, respectively alone or in combination, contribute to solving the object of the invention.
(1) A process for the manufacture of a compound of formula 1 or 1' 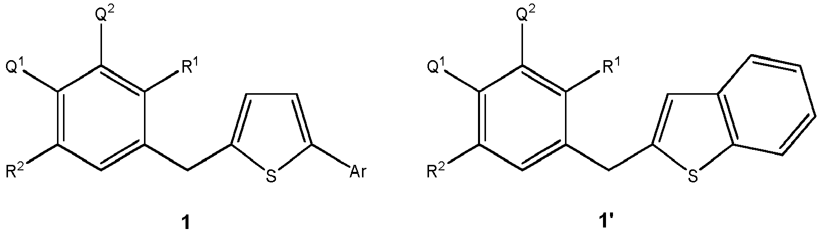
   wherein R¹ represents hydrogen, halo, C₁-C₆-alkyl, hydroxy, C₁-C₆-alkoxy, thio, C₁-C₆-alkylthio, C₁-C₆-alkylcarbonylamino, or C₁-C₆-alkanesulfonylamino;
   R² represents halo, C₁-C₆-alkyl, nitro, cyano, carboxy, or C₁-C₆-alkoxycarbonyl; and Q¹ and Q² are selected from hydrogen, or represent a condensed ring selected from benzo, pyrrolo[b], furano[b], thieno[b] or imidazo[d];
   wherein Ar is selected from phenyl, 1-naphthyl, 2-naphthyl, 3-pyridyl, 5-pyrimidinyl, 2-indolyl or 3-indolyl, which is optionally substituted by one or more substituents selected from fluoro, chloro, C₁-C₆-alkyl, C₃-C₇-cycloalkyl, phenyl, hydroxy, C₁-C₆-alkoxy, cyano, carboxy, C₁-C₆-alkoxycarbonyl, C₁-C₆-alkylcarbonyl, C₁-C₆-alkanesulfonyl, C₆-C₁₂-arenesulfonyl, C₁-C₆-alkylthio, amino, optionally mono- or disubstituted with C₁-C₆-alkyl, C₃-C₅-alkyleneamino, 1-piperazinyl, nitro, azido, or -SiA¹A²A³, wherein A¹, A², and A³ are selected from C₁-C₆-alkyl, C₃-C₇-cycloalkyl, phenyl, or C₁-C₆-alkoxy; and
   wherein C₁-C₆-alkyl itself and each C₁-C₆-alkyl of selected groups, C₃-C₇-cycloalkyl, phenyl, C₃-C₅-alkyleneamino, 1-piperazinyl are optionally further substituted by one or more groups selected from C₁-C₄-alkyl, C₁-C₄-alkoxy, di-C₁-C₆-alkylamino, C₃-C₅-alkyleneamino phenyl, 1-piperazinyl, fluoro, cyano, or nitro;
   said process comprising the steps of:
   a) converting a compound of formula **5** or **5'** into a compound of formula **6** or **6'** 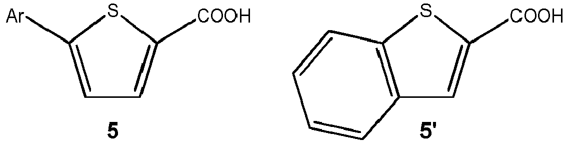 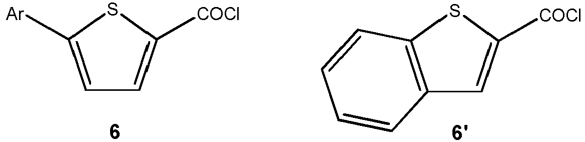 wherein Ar is defined as above;
   b) reacting the compound of formula **6** or **6'**
      with a compound of formula **7** 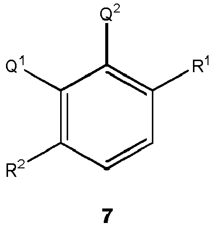 wherein R¹, R², Q¹ and Q² are defined as above;
      to obtain a compound of formula 8 or 8' 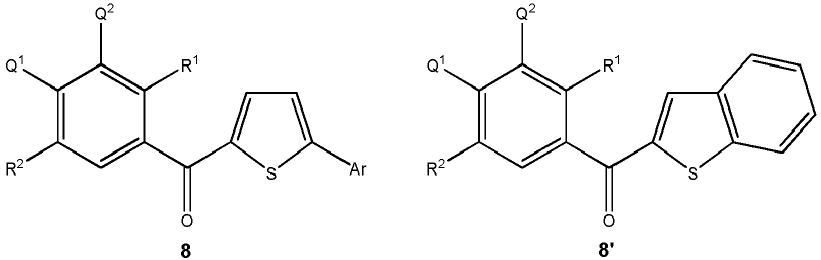 wherein R¹, R², Q¹, Q² and Ar are defined as above; and
   c) reducing the compound of formula **8** or **8'**
      to obtain the compound of formula **1** or **1'**.
(2) The process according to item 1, wherein step a) is conducted in the presence of a chlorinating agent.
(3) The process according to item 1 or item 2, wherein the chlorinating agent is SOCl₂, oxalyl chloride, PCl₃ or PCl₅.
(4) The process according to any one of preceding items, wherein step b) is conducted in the presence of a Lewis acid.
(5) The process according to item 4, wherein the Lewis acid is FeCl₃, ZnCl₂ or AlCl₃.
(6) The process according to any one of preceding items, wherein step b) is conducted in a solvent such as halogenated alkanes, alkanes, ethers or CS₂.
(7) The process according to item 6, wherein step b) is conducted in dichloromethane.
(8) The process according to any one of preceding items, wherein step b) is conducted at a temperature from ambient to the boiling point of the solvent.
(9) The process according to any one of preceding items, wherein the compound of formula **8** or **8'** is reduced with triethylsilane in the presence of borontrifluoride etherate.
(10) The process according to any one of preceding items, wherein the compound of formula **5** is prepared by a process comprising the steps of:
   a) reacting a compound of formula **2**

      **Ar-X** **2**

      wherein X is halo, and Ar is defined as above,
      with a compound of formula 3 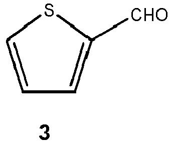 to obtain a compound of formula **4** 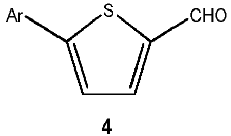 wherein Ar is defined as above; and
   b) oxidizing the compound of formula **4**
      to obtain the compound of formula **5.**
(11) The process according to item 10, wherein X is Br, Cl or I, preferably Br.
(12) The process according to item 10 or item 11, wherein step a) is conducted in the presence of palladium catalyst, preferably palladium acetate.
(13) The process according to any one of items 1 to 9, wherein the compound of formula **5'** is prepared by oxidizing the compound of formula **4'** 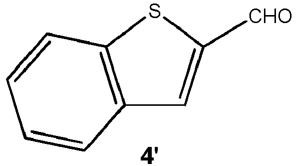 to obtain the compound of formula **5'.**
(14) The process according to any one of items 10 to 13, wherein the oxidation in step b) is conducted in the presence of an oxidizing agent selected from the group consisting of sodium chlorite, sodium perchlorate, sodium hypochlorite, potassium dichromate, oxone, sodium perborate, pyridinium chlorochromate, periodic acid and potassium permanganate.
(15) The process according to any one of preceding items, wherein Q¹ and Q² respectively represent hydrogen.
(16) The process according to any one of preceding items, wherein Ar is phenyl, which is optionally substituted by one or more substituents selected from fluoro, chloro, C₁-C₆-alkyl, C₃-C₇-cycloalkyl, phenyl, hydroxy, C₁-C₆-alkoxy, cyano, carboxy, C₁-C₆-alkoxycarbonyl, C₁-C₆-alkylcarbonyl, C₁-C₆-alkanesulfonyl, C₆-C₁₂-arenesulfonyl, C₁-C₆-alkylthio, amino, optionally mono- or disubstituted with C₁-C₆-alkyl, C₃-C₅-alkyleneamino, 1-piperazinyl, nitro, azido, or -SiA¹A²A³, wherein A¹, A², and A³ are selected from C₁-C₆-alkyl, C₃-C₇-cycloalkyl, phenyl, or C₁-C₆-alkoxy; and wherein C₁-C₆-alkyl itself and each C₁-C₆-alkyl of selected groups, C₃-C₇-cycloalkyl, phenyl, C₃-C₅-alkyleneamino, 1-piperazinyl are optionally further substituted by one or more groups selected from C₁-C₄-alkyl, C₁-C₄-alkoxy, di-C₁-C₆-alkylamino, C₃-C₅-alkyleneamino phenyl, 1-piperazinyl, fluoro, cyano, or nitro.
(17) The process according to item 16, wherein Ar is halo-substituted phenyl, preferably phenyl para-substituted by fluoro.
(18) The process according to any one of preceding items, wherein R¹ is C₁-C₆-alkyl, preferably CH₃, or halo, preferably fluoro; more preferably R¹ is CH₃.
(19) The process according to any one of preceding items, wherein R² is Br, I or Cl, preferably Br.
(20) The process according to any one of preceding items, further comprising coupling the compound of formula **1** or **1**' with a glucose or its derivative moiety to give a C-aryl glycoside compound.
(21) The process according to item 20, comprising the steps of:
   a) coupling the compound of formula **1** or **1**'
      with a hydroxyl-protected glucose or its derivative, and
   b) removing protecting groups from the resulting compound
      to obtain a C-aryl glycoside compound.
(22) Use of the compound of formula **1** or **1'** 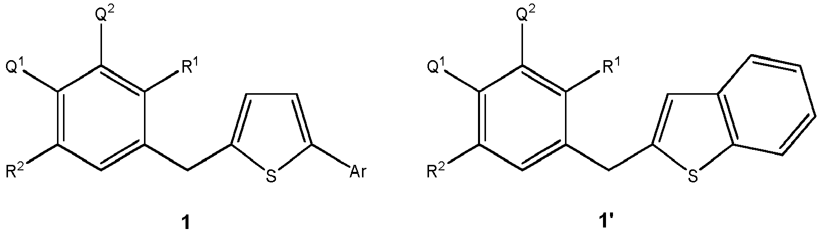 wherein R¹, R², Q¹, Q² and Ar are as defined in item 1,
   for the preparation of C-aryl glycosides.
(23) A process for the preparation of a pharmaceutical composition comprising a C-aryl glycoside compound, comprising the steps of:
   a) preparing a C-aryl glycoside compound by carrying out a process according to item 20 or item 21, and
   b) mixing the C-aryl glycoside compound with a pharmaceutically acceptable carrier and/or excipient.
(24) The process or use according to any one of items 20 to 23, wherein the C-aryl glycoside compound is a compound of formula **I** (canagliflozin) 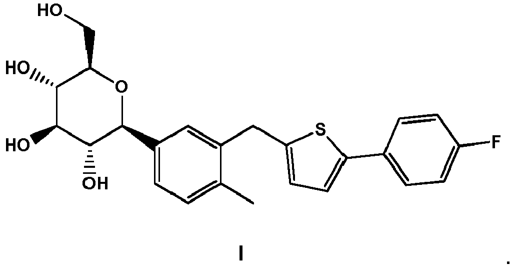
(25) The process or use according to any one of items 20 to 23, wherein the C-aryl glycoside compound is a compound of formula **II** (ipragliflozin)

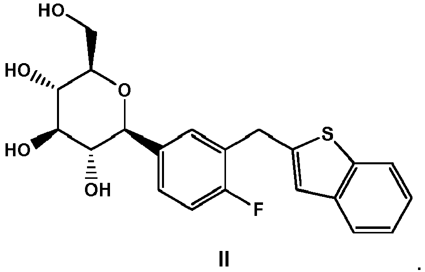

The new process defined above provides 2-arylmethyl-5-aryl-thiophene in a shorter and more efficient way without the need of using expensive materials and reagents, which provides an industrially preferable and economically improved process using said compounds as intermediates.

### Detailed Description of the Invention

The present invention is now described in more detail by preferred embodiments and examples, which are however presented for illustrative purpose only.

A general concept of the process of the present invention may be represented in **Scheme 1.**

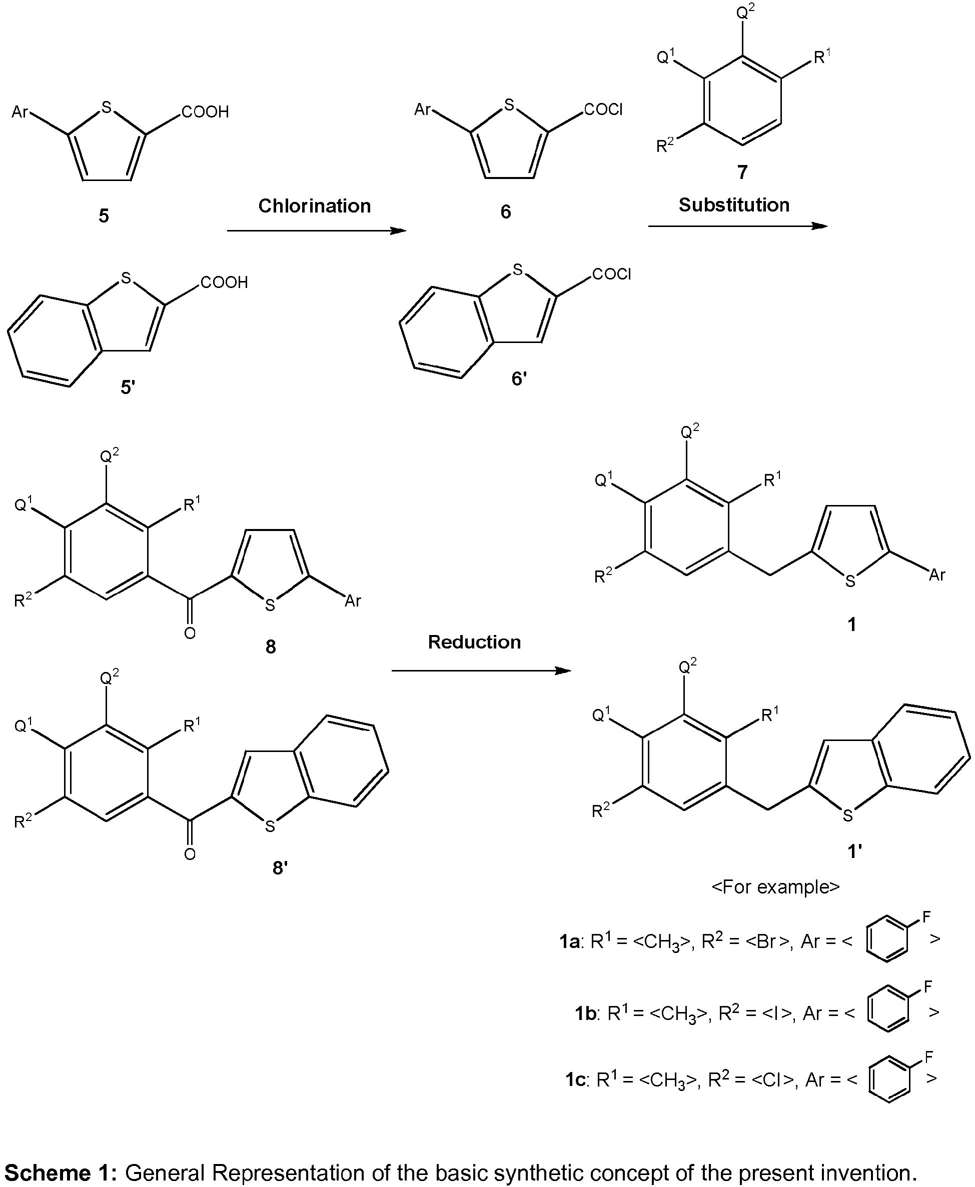

In the first step, the compound of formula **5** or **5'** is converted into a derivate suitable for the following Friedel-Crafts reaction, acyl chloride compound of formula **6** or **6'.** Any known method to form a derivative suitable for Friedel-Crafts reaction can be used for this step, for example, but not limited to, thionyl chloride, oxalyl chloride, phosphorus trichloride or phosphorus pentachloride can be used.

In the next step, the compound of formula **6** or **6'** is reacted with a compound of formula **7,** stoichiometrically or with excess of the compound of formula **7,** to give a compound of formula **8** or **8'.** In a specific embodiment of the present invention the reaction of the compound of formula **6** or **6'** and the compound of formula **7** is conducted in the presence of an appropriate Lewis acid, for example, FeCl₃, ZnCl₂ or AlCl₃, in an appropriate solvent such as halogenated alkanes, alkanes, ethers or CS₂, for example in dichloromethane. Preferably, the reaction can be carried out either at ambient temperature or heated to any temperature from ambient to the boiling point of the solvent.

In the final step, the compound of formula **8** or **8'** is reduced to give the compound of formula **1** or **1**'. Any known reduction method can be used for this step, for example, but not limited to, triethylsilane in the presence of borontrifluoride etherate can be used.

In the present invention, R¹ represents hydrogen, halo, C₁-C₆-alkyl, hydroxy, C₁-C₆-alkoxy, thio, C₁-C₆-alkylthio, C₁-C₆-alkylcarbonylamino, or C₁-C₆-alkanesulfonylamino; and Q¹ and Q² are selected from hydrogen, or represent a condensed ring selected from benzo, pyrrolo[b], furano[b], thieno[b] or imidazo[d]. Preferably, R¹ is C₁-C₆-alkyl, in particular CH₃, or halo, in particular fluoro. R² is halo, C₁-C₆-alkyl, nitro, cyano, carboxy, or C₁-C₆-alkoxycarbonyl, in a more preferred embodiment of the present invention R² is Br, I or Cl. In a most preferred embodiment of the present invention R² is Br. R² is preferably selected from groups which can be used in the coupling reaction with an appropriate glucose or its derivative, and in a particular embodiment of the present invention R² is a group capable of reacting or exchanging with metals such as, but not limited to, magnesium, lithium, copper, aluminum, titanium, palladium, zinc, and so on, in particular halogen.

In a further embodiment of the present invention Q¹ and Q² respectively represent hydrogen.

In the present invention, Ar is selected from phenyl, 1-naphthyl, 2-naphthyl, 3-pyridyl, 5-pyrimidinyl, 2-indolyl or 3-indolyl, which is optionally substituted by one or more substituents selected from fluoro, chloro, C₁-C₆-alkyl, C₃-C₇-cycloalkyl, phenyl, hydroxy, C₁-C₆-alkoxy, cyano, carboxy, C₁-C₆-alkoxycarbonyl, C₁-C₆-alkylcarbonyl, C₁-C₆-alkanesulfonyl, C₆-C₁₂-arenesulfonyl, C₁-C₆-alkylthio, amino, optionally mono- or disubstituted with C₁-C₆-alkyl, C₃-C₅-alkyleneamino, 1-piperazinyl, nitro, azido, or -SIA¹A²A³, wherein A¹, A², and A³ are selected from C₁-C₆-alkyl, C₃-C₇-cycloalkyl, phenyl, or C₁-C₆-alkoxy. Here, C₁-C₆-alkyl itself and each C₁-C₆-alkyl of selected groups, C₃-C₇-cycloalkyl, phenyl, C₃-C₅-alkyleneamino, 1-piperazinyl are optionally further substituted by one or more groups selected from C₁-C₄-alkyl, C₁-C₄-alkoxy, di-C₁-C₆-alkylamino, C₃-C₅-alkyleneamino phenyl, 1-piperazinyl, fluoro, cyano, or nitro.

In a preferred embodiment of the present invention Ar is phenyl, which is optionally substituted by one or more substituents defined as above. More preferably, Ar is halo-substituted phenyl, in particular phenyl para-substituted by fluoro.

In a particular embodiment, the compound of formula 5 may be prepared by a reaction illustrated in Scheme 2.

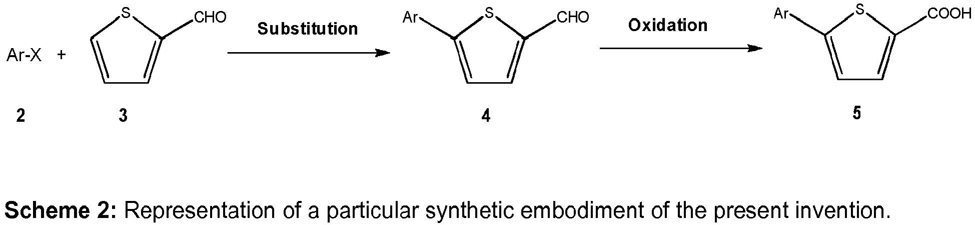

In the first step, the compound of formula 4 is prepared by reacting the compound of formula 2 with the compound of formula 3. Ar and X are as defined above. In a preferred embodiment of the present invention the coupling reaction is conducted in the presence of palladium catalyst, preferably palladium acetate.

In another particular embodiment, the compound of formula 5' is prepared by a reaction illustrated in **Scheme 3.**

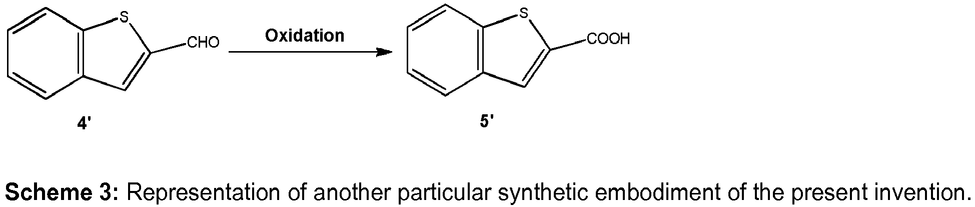

The compound of formula **4'** can be produced as described, for example, in Podea, Paula Veronica et al, Tetrahedron: Asymmetry, 19(4), 500-511; 2008.

In the next step, the aldehyde compound of formula **4** or **4'** is oxidized to give the carboxylic acid compound of formula **5** or **5'.** Any known oxidation method can be used for this step, for example, but not limited to, sodium chlorite, sodium perchlorate, sodium hypochlorite, potassium dichromate, oxone, sodium perborate, pyridinium chlorochromate, periodic acid and potassium permanganate can be used.

In a further aspect of the present invention C-aryl glycosides may be prepared by coupling the compound of formula **1** with an appropriate glucose or its derivative. Typically, the hydroxyl groups of glucose or its derivative may be protected with suitable protecting groups, which may be subsequently removed after the coupling reaction with the aryl compound. Here, protecting groups mean a group which may be attached to an oxygen atom to protect said oxygen atom from participating in a reaction and which may be readily removed following the reaction. Suitable protecting groups include, but are not limited to, ethers such as benzyl, *para-*methoxybenzyl or other substituted benzyl ethers, aryl, alkyl; esters such as acetyl, pivaloyl, benzoyl and other alkyl or aryl esters; silyl ethers such as trimethylsilyl, tert-butyldimethylsilyl and similar or any other suitable protecting groups. Other suitable protecting groups may be found in texts such as T.W. Greene & P. G. M. Wuts, Protective Groups in Organic Synthesis, John Wiley & Sons, 1991.

In a particular embodiment of such a further aspect of the present invention, the compound of formula 1 or **1**' is effectively used for the preparation of particularly selected C-aryl glycosides, preferably β-C-aryl glycosides. Preferred examples of β-C-aryl glycosides include a compound of formula I (canagliflozin) and a compound of formula II (ipragliflozin).

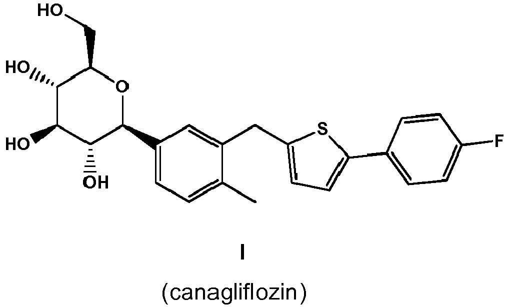

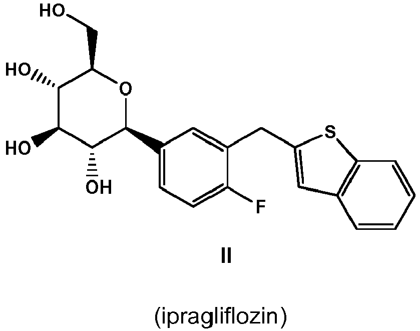

In the present invention the coupling reaction herein may be carried out in accordance with a suitable coupling reaction mechanism, for instance in the presence of a metal catalyst. Examples of a metal catalyst may include, but are not limited to, palladium, nickel, copper, cobalt, and so on. Exemplary coupling reactions may include, but are not limited to, the coupling of a hydroxyl-protected glucose or its derivative such as gluconolactone or glucal with a metalated aryl derivative, or of a metalated glucose or its derivative with an aryl halide, or of a hydroxyl-protected glycosyl halide with a metalated aryl compound.

In a preferred embodiment of the present invention the compound of formula **1** is lithiated with a suitable alkyl lithium such as tert-butyl lithium, n-butyl lithium, and so on, in a suitable solvent such as diethyl ether, tetrahydrofuran, and so on, and subsequently, reacted with a gluconolactone derivative.

The C-aryl glycoside compound, which is prepared according to the invention, notably canagliflozin and ipragliflozin, can be beneficially used or administered preferably as a pharmaceutical composition comprising the C-aryl glycoside compound and a pharmaceutically acceptable excipient and/or carrier. Further, the C-aryl glycoside compound prepared according to the invention may be used or administered on its own or combined with other drugs, especially with drugs used for the normalization of plasma glucose levels, for example for the treatment or the prophylaxis of diabetes mellitus.

In a further aspect of the present invention, a pharmaceutical composition comprising the C-aryl glycoside compound is prepared by comprising the steps of preparing the C-aryl glycoside compound, as described above, and mixing the C-aryl glycoside compound with a pharmaceutically acceptable carrier and/or excipient. The administration form can be suitably chosen, for example, a form suitable for oral, parenteral, rectal administration and/or administration by inhalation, and the dosage form may be solid, liquid or powdery. Therefore, the pharmaceutical composition comprising the C-aryl glycoside compound prepared according to the invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the compound or a finely divided form thereof, or particles comprising the compound, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The compound or a finely divided form thereof, or particles containing the same may be coated by another substance. The powder mixture or particles containing the compound may also be dispensed into capsules.

The pharmaceutical composition comprising the C-aryl glycoside compound prepared according to the invention in a desired dose is generally suitable to treat or prevent a disease or condition of a patient in need thereof, specifically to display a desired activity when lowering plasma glucose levels, for example treating or preventing diabetes mellitus.

In a preferred embodiment of the present invention the C-aryl glycoside compound is a compound of formula **I** (canagliflozin) or a compound of formula **II** (ipragliflozin). Particularly preferred is the preparation of canagliflozin.

The term "C-aryl glycoside" as employed herein may include any salts, hydrates and solvates thereof, and crystalline forms thereof.

The following Examples are non-limiting and serve to illustrate the invention.

### Examples

### Example 1: Preparation of 5-(4-fluoro-phenyl)-thiophene-2-carbaldehyde (4)

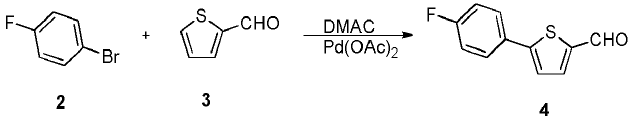

4-Bromo-1-fluoro-benzene (**2**, 33 ml) and thiophene carbaldehyde (3, 56 ml), potassium acetate (59 g) and palladium acetate (66 mg) were dissolved in dimethylacetamide (500 ml) and heated at 140 - 150 °C for 22 h. The reaction mixture was concentrated and water (300 ml) was added. Resulting precipitate was washed with water and recrystallized from 2-propanol to give 35.4 g of the title compound **4**. ¹³C NMR (CDCl₃): 116.25, 116.42, 124.10, 128.28, 128.35, 137.46, 142.53, 153.06, 164.43, 164.42, 182.77.

### Example 2: Preparation of 5-(4-fluoro-phenyl)-thiophene-2-carboxylic acid (5)

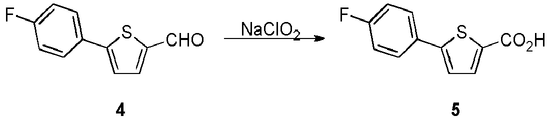

5-(4-Fluoro-phenyl)-thiophene-2-carbaldehyde (4, 5.44 g) was dissolved in acetonitrile (50 ml). NaH₂PO₄ (0.92 g) in water (11 ml) was added to the reaction mixture and 30% hydrogen peroxide (2.75 ml) was further added thereto. A solution of NaClO₂ (4.20 g) in water (25 ml) was slowly added for 30 mins. The reaction mixture was heated to 40 °C and stirred at that temperature for another hour. Precipitate was collected by filtration to give 6.21 g of the title compound **5.** ¹³C NMR (CDCl₃): 116.18, 116.35, 124.69, 128.04, 128.11, 128.59, 129.57, 134.06, 134.17, 148.26, 161.36, 162.98, 163.32.

### Example 3: Preparation of 5-(4-fluoro-phenyl)-thiophene-2-carbonyl chloride (6)

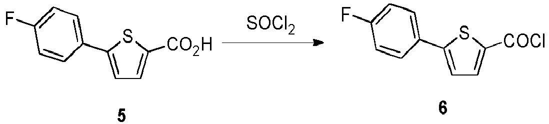

5-(4-Fluoro-phenyl)-thiophene-2-carboxylic acid (**5**, 5.0 g) was dissolved in toluene (50 ml) and thionyl chloride (3.26 ml) was added. The mixture was heated at 90 °C for 20 h after which another 1 ml of thionyl chloride was added and heated for another hour at 90 °C. The reaction mixture was evaporated to give 5.34 g of the title compound **6.** MS, m/z= 240. ¹H NMR (acetone): 7.29 (m, 2H); 7.66 (d, 1 H), 7.88 (m, 2H), 8.07 (d, 1 H).

### Example 4: Preparation of 2-(5-bromo-2-methyl-benzoyl)-5-(4-fluoro-phenyl)-phenyl)-thiophene (8)

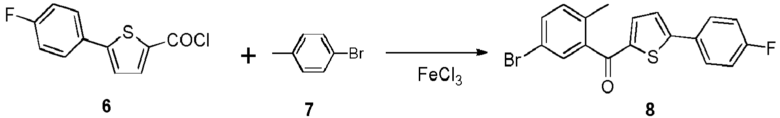

5-(4-Fluoro-phenyl)-thiophene-2-carbonyl chloride (**6**, 1.20 g) and 4-bromo-toluene (**7**, 9.85 g) were dissolved in dichloromethane (10 ml), and then FeCl₃ (0.97 g) was added. The reaction mixture was stirred at room temperature for 20 h. 2M HCl (10 ml) was added and phases were separated. Organic phase was washed with 2M HCl (3 x 10 ml) and subsequently with brine (30 ml), and dried over sulfate and then concentrated. Raw material was purified with column chromatography (ethyl acetate/methylcyclohexane) to give 0.77 g of the title compound **8.** ¹³C NMR (CDCl₃): 19.41, 116.41, 116.59, 119.01, 124.33, 128.40, 128.47, 129.67, 130.70, 132.95, 133.37, 135.59, 137.05, 140.22, 142.89, 153.55, 162.55, 164.54, 188.62.

### Example 5: Preparation of 2-(5-bromo-2-methyl-benzyl)-5-(4-fluoro-phenyl)-thiophene (1 a)

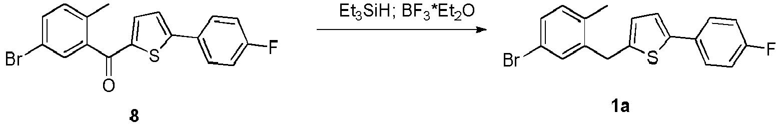

2-(5-Bromo-2-methyl-benzoyl)-5-(4-fluoro-phenyl)-phenyl)-thiophene (**8**, 3.75 g) was dissolved in dichloromethane (40 ml) and acetonitrile (40 ml), to which triethylsilane (4.63 ml) was added and cooled to 0 °C. Boron trifluoride etherate (3.45 ml) was added to the suspension and the reaction mixture was left to warm up to room temperature then continued to stir at that temperature for 26 h. The reaction mixture was cooled on ice and 50 ml of NaHCO₃ was slowly added, followed by adding dichloromethane (50 ml). Phases were separated and organic phase was dried over sulphate, and then evaporated. The residue was recrystallized from 2-propanol to give 3.65 g of the title compound **1a**. ¹³C NMR (DMSO): 18.52, 32.84, 115.83, 116.00, 118.81, 123.52, 126.86, 126.99, 127.05, 129.50, 130.41, 131.60, 132.37, 135.54, 140.63, 141.16, 142.46, 160.48, 162.42. MS, m/z= 360.

## Claims

1. A process for the manufacture of a compound of formula 1 or 1' 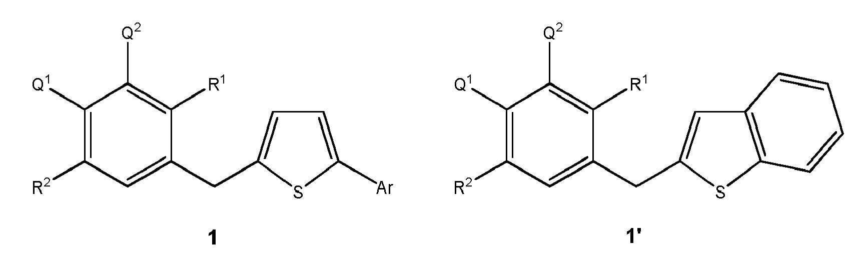
wherein R¹ represents hydrogen, halo, C₁-C₆-alkyl, hydroxy, C₁-C₆-alkoxy, thio, C₁-C₆-alkylthio, C₁-C₆-alkylcarbonylamino, or C₁-C₆-alkanesulfonylamino;
R² represents halo, C₁-C₆-alkyl, nitro, cyano, carboxy, or C₁-C₆-alkoxycarbonyl; and
Q¹ and Q² are selected from hydrogen, or represent a condensed ring selected from benzo, pyrrolo[b], furano[b], thieno[b] or imidazo[d];
wherein Ar is selected from phenyl, 1-naphthyl, 2-naphthyl, 3-pyridyl, 5-pyrimidinyl, 2-indolyl or 3-indolyl, which is optionally substituted by one or more substituents selected from fluoro, chloro, C₁-C₆-alkyl, C₃-C₇-cycloalkyl, phenyl, hydroxy, C₁-C₆-alkoxy, cyano, carboxy, C₁-C₆-alkoxycarbonyl, C₁-C₆-alkylcarbonyl, C₁-C₆-alkanesulfonyl, C₆-C₁₂-arenesulfonyl, C₁-C₆-alkylthio, amino, optionally mono- or disubstituted with C₁-C₆-alkyl, C₃-C₅-alkyleneamino, 1-piperazinyl, nitro, azido, or -SiA¹A²A³, wherein A¹, A², and A³ are selected from C₁-C₆-alkyl, C₃-C₇-cycloalkyl, phenyl, or C₁-C₆-alkoxy; and
wherein C₁-C₆-alkyl itself and each C₁-C₆-alkyl of selected groups, C₃-C₇-cycloalkyl, phenyl, C₃-C₅-alkyleneamino, 1-piperazinyl are optionally further substituted by one or more groups selected from C₁-C₄-alkyl, C₁-C₄-alkoxy, di-C₁-C₆-alkylamino, C₃-C₅-alkyleneamino phenyl, 1-piperazinyl, fluoro, cyano, or nitro;
said process comprising the steps of:
a) converting a compound of formula **5** or **5'** into a compound of formula **6** or **6'** 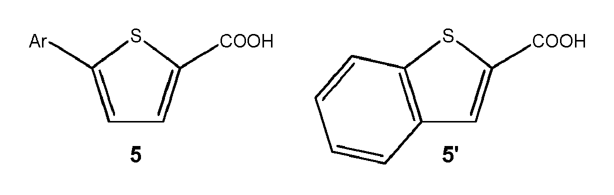 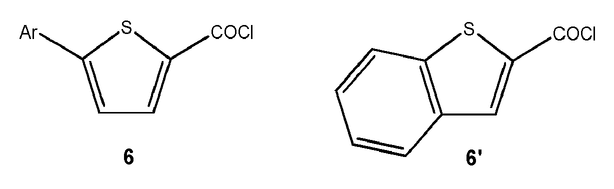 wherein Ar is defined as above;
b) reacting the compound of formula **6** or **6'**
with a compound of formula **7** 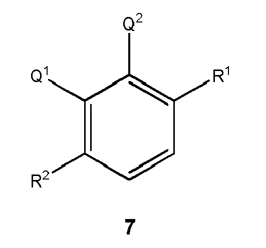 wherein R¹, R², Q¹ and Q² are defined as above;
to obtain a compound of formula 8 or 8' 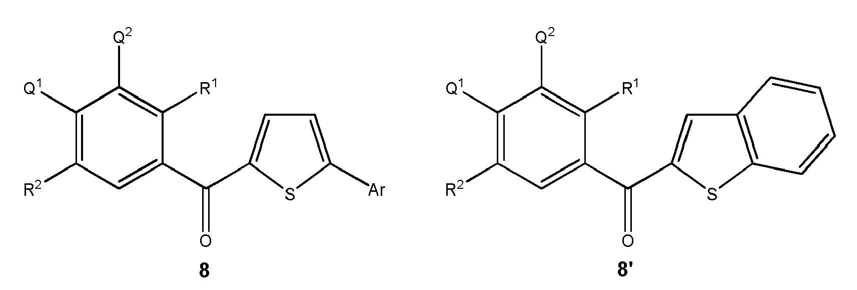 wherein R¹, R², Q¹, Q² and Ar are defined as above; and
c) reducing the compound of formula **8** or **8'**
to obtain the compound of formula **1** or **1**'.

2. The process according to claim 1, wherein step a) is conducted in the presence of a chlorinating agent, preferably SOCl₂, oxalyl chloride, PCl₃ or PCl₅.

3. The process according to any one of preceding claims, wherein step b) is conducted in the presence of a Lewis acid, preferably FeCl₃, ZnCl₂ or AlCl₃.

4. The process according to any one of preceding claims, wherein step b) is conducted in a solvent such as halogenated alkanes, alkanes, ethers or CS₂, preferably in dichloromethane.

5. The process according to any one of preceding claims, wherein step b) is conducted at a temperature from ambient to the boiling point of the solvent.

6. The process according to any one of preceding claims, wherein the compound of formula **8** or **8'** is reduced with triethylsilane in the presence of borontrifluoride etherate.

7. The process according to any one of preceding claims, the compound of formula **5** or **5'** is prepared by a process comprising the steps of
a1) reacting a compound of formula **2**
**Ar-X** **2**
wherein X is halo, and Ar is defined as above,
with a compound of formula 3 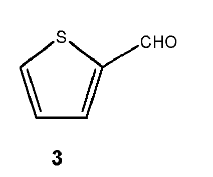 to obtain a compound of formula **4** 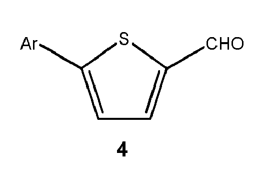 wherein Ar is defined as above; or
a2) providing a compound of formula **4'** 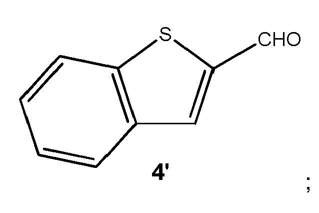 and
b) oxidizing the compound of formula **4** or **4'**
to obtain the compound of formula **5** or **5'.**

8. The process according to claim 7, wherein X is Br, Cl or I, preferably Br.

9. The process according to claim 7 or 8, wherein step a1) is conducted in the presence of palladium catalyst, preferably palladium acetate.

10. The process according to any one of claims 7 to 9, wherein the oxidation in step b) is conducted in the presence of an oxidizing agent selected from the group consisting of sodium chlorite, sodium perchlorate, sodium hypochlorite, potassium dichromate, oxone, sodium perborate, pyridinium chlorochromate, periodic acid and potassium permanganate.

11. The process according to any one of preceding claims, wherein Q¹ and Q² respectively represent hydrogen; or
wherein Ar is phenyl, which is optionally substituted by one or more substituents selected from fluoro, chloro, C₁-C₆-alkyl, C₃-C₇-cycloalkyl, phenyl, hydroxy, C₁-C₆-alkoxy, cyano, carboxy, C₁-C₆-alkoxycarbonyl, C₁-C₆-alkylcarbonyl, C₁-C₆-alkanesulfonyl, C₆-C₁₂-arenesulfonyl, C₁-C₆-alkylthio, amino, optionally mono- or disubstituted with C₁-C₆-alkyl, C₃-C₅-alkyleneamino, 1-piperazinyl, nitro, azido, or -SiA¹A²A³, wherein A¹, A², and A³ are selected from C₁-C₆-alkyl, C₃-C₇-cycloalkyl, phenyl, or C₁-C₆-alkoxy; and wherein C₁-C₆-alkyl itself and each C₁-C₆-alkyl of selected groups, C₃-C₇-cycloalkyl, phenyl, C₃-C₅-alkyleneamino, 1-piperazinyl are optionally further substituted by one or more groups selected from C₁-C₄-alkyl, C₁-C₄-alkoxy, di-C₁-C₆-alkylamino, C₃-C₅-alkyleneamino phenyl, 1-piperazinyl, fluoro, cyano, or nitro; preferably halo-substituted phenyl, more preferably phenyl para-substituted by fluoro; or
wherein R¹ is C₁-C₆-alkyl, preferably CH₃, or halo, preferably fluoro; more preferably CH₃; or R² is Br, I or Cl, preferably Br.

12. The process according to any one of preceding claims, further comprising coupling the compound of formula **1** or **1**' with a glucose or its derivative moiety to give a C-aryl glycoside compound; preferably comprising the steps of:
a) coupling the compound of formula **1** or **1**'
with a hydroxyl-protected glucose or its derivative, and
b) removing protecting groups from the resulting compound
to obtain a C-aryl glycoside compound.

13. Use of the compound of formula **1** or **1'** 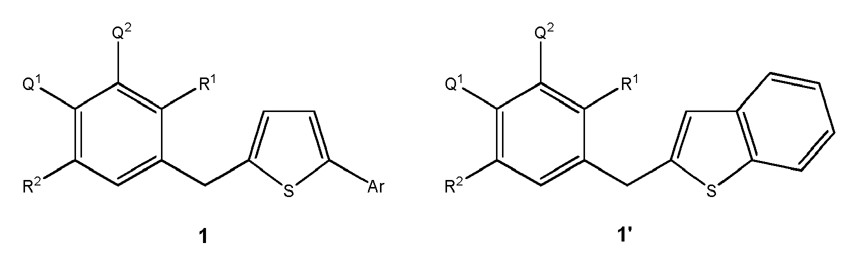 wherein R¹, R², Q¹, Q² and Ar are as defined in claim 1,
for the preparation of C-aryl glycosides.

14. A process for the preparation of a pharmaceutical composition comprising a C-aryl glycoside compound, comprising the steps of:
a) preparing a C-aryl glycoside compound by carrying out a process according to claim 12, and
b) mixing the C-aryl glycoside compound with a pharmaceutically acceptable carrier and/or excipient.

15. The process or use according to any one of claims 12 to 14, wherein the C-aryl glycoside compound is a compound of formula **I** (Canagliflozin) 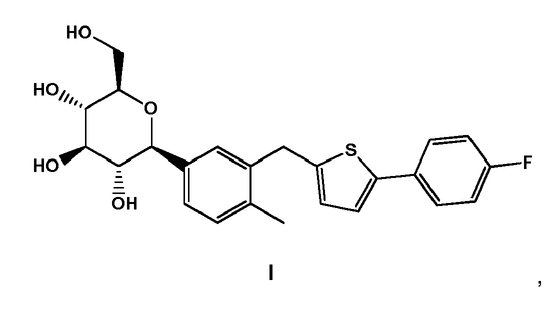 or
a compound of formula **II** (Ipragliflozin)
